# EUROPÄISCHE PATENTSCHRIFT

(11) **EP 4 486 276 B1**
(45) Veröffentlichungstag und Bekanntmachung des Hinweises auf die Patenterteilung: **31.12.2025**
(21) Anmeldenummer: 23705336.8
(22) Anmeldetag: 31.01.2023
(51) Int. Cl.: A61F 5/01, A61F 13/06, A61F 5/30, A61F 13/10

(54) **VERFAHREN ZUM BEFESTIGEN EINER PELOTTE AN EINEM ELASTISCHEN GESTRICKKÖRPER, VERFAHREN ZUM HERSTELLEN EINER BANDAGE, GESTRICKKÖRPER MIT DARAN BEFESTIGTER PELOTTE UND BANDAGE**
METHOD FOR FASTENING A TRUSS PAD TO AN ELASTIC KNITTED BODY, METHOD FOR PRODUCING A DRESSING, KNITTED BODY HAVING A TRUSS PAD FASTENED THERETO, AND DRESSING
PROCÉDÉ DE FIXATION D'UN TAMPON EN TREILLIS SUR UN CORPS TRICOTÉ ÉLASTIQUE, PROCÉDÉ DE FABRICATION D'UN PANSEMENT, CORPS TRICOTÉ SUR LEQUEL EST FIXÉ UN TAMPON EN TREILLIS, ET PANSEMENT

(30) Priorität: 28.02.2022 DE 102022104686
(43) Veröffentlichungstag der Anmeldung: 08.01.2025
(73) Patentinhaber: Thuasne Deutschland GmbH, 30539 Hannover (DE)
(72) Erfinder: SCHNEIDER-NIESKENS, Reinhold, 29224 Celle (DE)
(74) Vertreter: Jabbusch, Matthias
(86) Internationale Anmeldenummer: PCT/EP2023/052290
(87) Internationale Veröffentlichungsnummer: WO 2023/160967

(56) Entgegenhaltungen:
- EP-B1- 0 728 454
- DE-U1- 202015 105 668

## Beschreibung

Die Erfindung betrifft ein Verfahren zum Befestigen einer Pelotte an einem elastischen Gestrickkörper einer herzustellenden Bandage nach dem Oberbegriff des Patentanspruchs 1. Die Erfindung betrifft weiter ein Verfahren zum Herstellen einer Bandage, welches das erfindungsgemäße Verfahren zum Befestigen der Pelotte am elastischen Gestrickkörper beinhaltet. Außerdem betrifft die Erfindung einen Gestrickkörper mit daran befestigter Pelotte nach dem Oberbegriff des Patentanspruchs 11. Schließlich betrifft die Erfindung auch eine Bandage, die den hier schlauchförmigen erfindungsgemäßen Gestrickkörper mit daran befestigter Pelotte aufweist. Die Bandage ist insbesondere eine Extremitätenbandage und zur Anlage an ein Gelenk wie Kniegelenk, Fußgelenk, Ellenbogengelenk oder Handgelenk bestimmt.

Bei der Herstellung der Bandage wird die Pelotte zwischen dem Gestrickkörper und einer Pelottenabdeckung angeordnet und wird die Pelottenabdeckung rings um die Kontur der Pelotte mit dem Gestrickkörper verbunden, so dass die Pelotte vollständig von dem Gestrickkörper und der Pelottenabdeckung umschlossen wird. Die Pelottenabdeckung soll bei der späteren Verwendung der Bandage in der Regel an der Extremität anliegen, wohingegen der Gestrickkörper die Pelottenabdeckung außen umschließen soll.

Traditionell kann die Pelottenabdeckung mittels Nähten mit der Bandage verbunden werden. Die Nähte können allerdings bei längerem Gebrauch der Bandage durchscheuern und stören außerdem das äußere Erscheinungsbild der Bandage. Es ist daher bekannt, die Pelottenabdeckung alternativ mittels einer Klebeverbindung in Form einer großflächig auf den Gestrickkörper oder die Pelottenabdeckung aufgebrachten Klebstoffschicht mit dem Gestrickkörper zu verbinden und dabei auch die Pelotte mit dem Gestrickkörper oder der Pelottenabdeckung zu verbinden. Die Klebstoffschicht ist dabei in der Regel aus einem Schmelzkleber gebildet.

Es hat sich gezeigt, dass eine derartige Klebeverbindung zu ähnlichen Kosten herstellbar ist wie Nähte. Von Vorteil bei diesem bekannten Verfahren zum Befestigen einer Pelotte an einem elastischen Gestrickkörper ist außerdem, ihre Dauerhaftigkeit, da keine Nähte durchscheuern können, und das äußere Erscheinungsbild der entsprechend hergestellten Bandage. Von Nachteil sind hingegen die mangelnde Dehnbarkeit und die mangelnde Dampfdurchlässigkeit der entsprechend hergestellten bekannten Bandage. Dadurch wird der Tragekomfort beeinträchtigt. Insbesondere blockieren bei derartigen bekannten Bandagen die Pelotten mit ihren relativ unelastischen mit Schmelzkleber beschichteten Abdecktextilien die Dehnbarkeit der Bandagen in Umfangsrichtung. Diese verminderte Dehnbarkeit erschwert das Anziehen der Bandagen und erhöht die Faltenbildung bei Gelenkbeugung.

Der Erfindung liegt die Aufgabe zugrunde, ein Verfahren zum Befestigen einer Pelotte an einem elastischen Gestrickkörper einer herzustellenden Bandage, ein Verfahren zum Herstellen der Bandage, einen verfahrensgemäß hergestellten Gestrickkörper mit der daran befestigen Pelotte und eine verfahrensgemäß hergestellte Bandage anzugeben, wobei die Bandage einen besonders hohen Tragekomfort aufweisen soll und zugleich langlebig sein soll und ein hochwertiges Erscheinungsbild haben soll.

Die Erfindung löst diese Aufgabe mit einem Verfahren zum Befestigen einer Pelotte an einem elastischen Gestrickkörper einer herzustellenden Bandage, insbesondere Extremitätenbandage, nach dem Patentanspruch 1. Die Erfindung löst diese Aufgabe außerdem mit einem Verfahren zum Herstellen einer Bandage, insbesondere Extremitätenbandage, nach dem Patentanspruch 10. Schutz ist außerdem für den verfahrensgemäß hergestellten Gestrickkörper mit daran befestigter Pelotte gemäß dem Patentanspruch 11 und für die Bandage, insbesondere Extremitätenbandage, nach dem Patentanspruch 12 beansprucht. Vorteilhafte Ausführungsformen der Erfindung sind in den Unteransprüchen angegeben.

Bei einem Verfahren zum Befestigen einer Pelotte an einem elastischen Gestrickkörper einer herzustellenden Bandage, insbesondere Extremitätenbandage, bei dem die Pelotte an den Gestrickkörper angelegt wird, bei dem eine Pelottenabdeckung auf der dem Gestrickkörper abgewandten Seite der Pelotte an die Pelotte angelegt wird und bei dem die Pelottenabdeckung rings um die Kontur der Pelotte mit dem Gestrickkörper verbunden wird, so dass die Pelotte vollständig von dem Gestrickkörper und der Pelottenabdeckung umschlossen wird, ist erfindungswesentlich vorgesehen, dass die Pelottenabdeckung zumindest rings um die Kontur der Pelotte gegen den Gestrickkörper gedrückt und zum Verbinden mit dem Gestrickkörper ein offenes Schmelzklebevlies, mit dem die Pelottenabdeckung ausgestattet ist, währenddessen erhitzt wird.

Bei dem Verfahren zum Herstellen einer Bandage, insbesondere Extremitätenbandage, mit einem elastischen Gestrickkörper und einer am Gestrickkörper befestigten Pelotte wird die Pelotte entsprechend am Gestrickkörper befestigt.

Bei einem Gestrickkörper mit daran befestigter Pelotte, bei dem die Pelotte am Gestrickkörper angelegt ist, bei dem eine Pelottenabdeckung auf der dem Gestrickkörper abgewandten Seite der Pelotte an die Pelotte angelegt ist und bei dem die Pelottenabdeckung rings um die Kontur der Pelotte mit dem Gestrickkörper verbunden ist, so dass die Pelotte vollständig von dem Gestrickkörper und der Pelottenabdeckung umschlossen ist, ist erfindungswesentlich die Pelotte mittels des erfindungsgemäßen Verfahrens und daher mittels eines offenen Schmelzklebevlieses, mit dem die Pelottenabdeckung ausgestattet ist, mit dem Gestrickkörper verbunden.

Die erfindungsgemäße Bandage, insbesondere Extremitätenbandage, weist den erfindungsgemäßen schlauchförmigen Gestrickkörper mit daran befestigter Pelotte auf.

Das Schmelzklebevlies schmilzt bei Erhitzung, verklebt dabei mit der Pelotte und dringt rings um die Kontur der Pelotte in den Gestrickkörper und in das Textil der Pelottenabdeckung ein, so dass eine dauerhaft feste Verbindung zwischen der Pelottenabdeckung und dem Gestrickkörper geschafften wird und die Pelottenabdeckung an der Pelotte fixiert wird. Dank der offenen Struktur des Schmelzklebevlieses bleibt das Schmelzklebevlies auch nach dem Erhitzen, Verkleben mit dem Gestrickkörper und Abkühlen dehnbar. Insbesondere ist das Schmelzklebevlies dehnbarer als eine vollflächige Beschichtung des Gestrickkörpers oder der Pelottenabdeckung mit Schmelzkleber. Damit bleibt der Gestrickkörper mit der Pelottenabdeckung insgesamt dehnbar.

Vorzugsweise ist auch die Pelotte dehnbar. Erfindungsgemäß ist vorgesehen, dass die Pelotte mit der Richtung ihrer größten Dehnbarkeit in Umfangsrichtung der herzustellenden Bandage zwischen dem Gestrickkörper und der Pelottenabdeckung angeordnet wird. In der Richtung ihrer größten Dehnbarkeit ist die Pelotte dabei vorzugsweise um ein Mehrfaches dehnbarer, insbesondere bei gleicher Zugkraft mindestens doppelt so weit dehnbar, wie senkrecht zu dieser Richtung. Die Richtung der größten Dehnbarkeit und die Richtung senkrecht hierzu sind dabei als jeweils lokal parallel zum Gestrickkörper ausgerichtet zu verstehen.

Die offene Struktur des Schmelzklebevlieses sorgt außerdem für eine hohe Dampfdurchlässigkeit der Pelottenabdeckung und daher bei entsprechender Dampfdurchlässigkeit der Pelotte für eine hohe Dampfdurchlässigkeit der Bandage. Vorzugsweise ist die Pelotte daher dampfdurchlässig.

Insbesondere für die Herstellung der Dehnbarkeit ist erfindungsgemäß vorgesehen, dass die Pelotte Außenkanäle auf der Seite, die dem Strickkörper zugewandt ist, aufweist, dass die Pelotte Lüftungskanäle auf der Seite, die der Pelottenabdeckung zugewandt ist, aufweist und dass die Lüftungskanäle zumindest teilweise parallel zu den Außenkanälen angeordnet sind. Dadurch ist lokal die Materialdicke der Pelotte gemindert, was eine leichtere Dehnbarkeit bewirkt. Vorzugsweise weist die Pelotte ihre größte Dehnbarkeit senkrecht zu den Außenkanälen auf.

Weiter ist bevorzugt vorgesehen, dass die Außenkanäle und die parallel zu den Außenkanälen angeordneten Lüftungskanäle abwechselnd zueinander, insbesondere zur Ausbildung einer Art Ziehharmonika-Struktur, angeordnet sind. Hierdurch ist die Dehnbarkeit der Pelotte noch einmal erhöht. Die Außenkanäle sind dabei bevorzugt in ungefähr 90° zur Umfangsrichtung der Bandage ausgerichtet, so dass sich die größte Dehnbarkeit der Bandage in ihrer Umfangsrichtung ergibt und dadurch das Anlegen der Bandage erleichtert ist.

Für die Dampfdurchlässigkeit der Pelotte ist bevorzugt vorgesehen, dass die Pelotte Durchbrüche aufweist, die in die Lüftungskanäle münden. Wasserdampf, der von der Haut abgegeben wird, kann so durch die Pelottenabdeckung in die Lüftungskanäle gelangen und durch die Lüftungskanäle zu den Durchbrüchen geleitet werden. Weiter durch den Gestrickkörper kann die Feuchtigkeit somit abgeleitet und der Tragekomfort erhöht werden.

Die Pelotte besteht vorteilhafterweise aus einem Elastomer, bevorzugt thermoplastischem Elastomer (TPE), insbesondere thermoplastischem Elastomer auf Urethanbasis (TPU), oder aus Silikon, insbesondere Silikonkautschuk.

Bei einer vorteilhaften Ausführungsform des erfindungsgemäßen Verfahrens, bei der der Gestrickkörper und die Pelotte und die Pelottenabdeckung mit dem Schmelzklebevlies jeweils dehnbar sind, werden der Gestrickkörper und die Pelotte und die Pelottenabdeckung mit dem Schmelzklebevlies mit der Richtung ihrer jeweiligen größten Dehnbarkeit parallel zueinander angeordnet und miteinander verbunden. Dadurch wird die größtmögliche Dehnbarkeit der Bandage insgesamt erreicht.

Der Gestrickkörper ist vorzugsweise schlauchförmig ausgebildet. Die Richtung der größten Dehnbarkeit des Gestrickkörpers ist dabei insbesondere seine Umfangsrichtung. Hierdurch ergibt sich auch für die Bandage die größte Dehnbarkeit in Umfangsrichtung.

Die Pelottenabdeckung besteht vorteilhafterweise aus einem Abdecktextil, insbesondere elastischem Frottee, und dem Schmelzklebevlies. Das Abdecktextil wird hierbei entweder bereits mit dem Schmelzklebevlies verbunden, insbesondere laminiert, an die Pelotte angelegt wird oder erst dann mit dem Schmelzklebevlies verbunden, wenn die Pelottenabdeckung mit dem Gestrickkörper verbunden wird.

Für das Erhitzen des Schmelzklebevlieses weist das Schmelzklebevlies polare Dielektrika, insbesondere Dipolmoleküle, auf. Das Schmelzklebevlies wird dann kapazitiv in einem elektrischen Wechselfeld erwärmt. Das Wechselfeld ist insbesondere ein Hochfrequenzfeld mit einer Frequenz im Bereich von 25 MHz bis 29 MHz, bevorzugt im Bereich von 26 MHz bis 28 MHz, besonderes bevorzugt von etwa 27 MHz.

Das Hochfrequenzfeld wird zwischen Elektroden ausgebildet, die bevorzugt aus Aluminium oder Messing bestehen. Bevorzugt ist dabei vorgesehen, dass wenigstens eine Elektrode auf die Pelottenabdeckung aufgelegt wird und dass das Schmelzklebevlies mit Hilfe der auf die Pelottenabdeckung aufgelegten Elektrode kapazitiv erwärmt wird. Gegenelektroden werden dabei bevorzugt von einer Unterlage gebildet.

Die Elektroden werden vorzugsweise mit Hilfe einer Schablone auf der Pelottenabdeckung positioniert. Die Schablone kann auch beim vorherigen Positionieren der Pelotte und der Pelottenabdeckung relativ zum Gestrickkörper helfen.

Bei einer bevorzugten Ausführungsform weist das Schmelzklebevlies ein Flächengewicht von mehr als 30 g/m², vorzugsweise mehr als 40 g/m², und weniger als 100 g/m², vorzugsweise weniger als 80 g/m², auf. Besonders bevorzugt beträgt das Flächengewicht des Schmelzklebevlieses in etwa 60 g/m². Das Flächengewicht des Schmelzklebevlieses ist damit deutlich geringer als das Flächengewicht bekannter vollflächiger Klebeflächen und bewirkt dennoch die Herstellung einer erstaunlich belastbaren Verbindung zwischen der Pelottenabdeckung und dem Gestrickkörper. Das Schmelzklebevlies hat eine offene Schmelzkleberstruktur und besteht bevorzugt aus Copolyamid.

Weitere Ausführungsformen der Erfindung ergeben sich aus den Patentansprüchen, aus den beigefügten Zeichnungen und aus der nachfolgenden Beschreibung eines in den Zeichnungen dargestellten besonders bevorzugten Ausführungsbeispiels der Erfindung. In den Zeichnungen zeigen:
- Figur 1:: eine Anordnung, bestehend aus einen Gestrickkörper, einer auf dem Gestrickkörper angeordneten Pelotte und einer lokal umgeschlagenen und die Pelotte daher nur unvollständig abdeckenden Pelottenabdeckung mit einem Schmelzklebevlies für eine erfindungsgemäße Extremitätenbandage gemäß einem Ausführungsbeispiel der Erfindung in einer Draufsicht;
- Figur 2:: eine weitere Pelotte für eine weitere erfindungsgemäße Extremitätenbandage in einer Draufsicht auf ihre zur Anlage an die Pelottenabdeckung bestimmte Innenseite (Figur 2a) und auf ihre zur Anlage an den Gestrickkörper bestimmte Außenseite (Figur 2b);
- Figur 3:: eine vereinfachte Seitenschnittansicht der weiteren Pelotte von Figur 2 in entspanntem Zustand (Figur 3a) und in gerecktem Zustand (Figur 3b);
- Figur 4:: eine Anordnung mit dem Gestrickkörper und der auf dem Gestrickkörper angeordnete Pelotte von Figur 1 in einer Draufsicht;
- Figur 5:: die Anordnung von Figur 1 mit dem Gestrickkörper und der auf dem Gestrickkörper angeordneten und die Pelotte vollständig abdeckenden Pelottenabdeckung von Figur 1 in einer Draufsicht;
- Figur 6:: eine Anordnung mit der auf eine flächige Elektrode einer Hochfrequenzheizeinrichtung aufgelegten Anordnung von Figur 5 und mit einer auf die Pelottenabdeckung aufgelegten Elektrode in perspektivischer Ansicht;
- Figur 7:: eine mit der Anordnung von Figur 6 hergestellte Extremitätenbandage, bei der die Ränder der Pelottenabdeckung mit dem Gestrickkörper verbunden sind, in einer Draufsicht;
- Figur 8:: eine Anordnung mit einem Gestrickkörper und einer auf den Gestrickkörper aufgelegten Schablone in einer Draufsicht;
- Figur 9:: eine auf der Anordnung von Figur 8 basierende Anordnung mit auf den Gestrickkörper aufgelegter und mittels der Schablone positionierter Pelotte und daneben angeordneten Klebestreifen in einer Draufsicht;
- Figur 10:: eine auf der Anordnung von Figur 9 basierende Anordnung mit über der Pelotte positionierter Pelottenabdeckung und über den Klebestreifen angeordneten Stützelementen in einer Draufsicht; und
- Figur 11:: eine auf der Anordnung von Figur 10 basierende Anordnung mit auf der Pelotte und den Stützelementen aufgelegten Elektroden in einer Draufsicht.

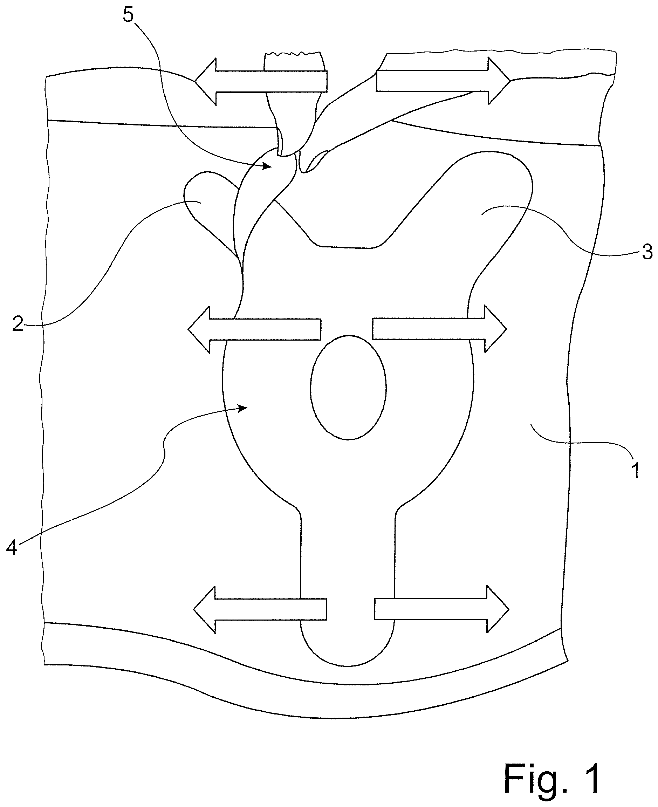

Figur 1 zeigt einen Ausschnitt von lose aufeinander gelegten Teilen, aus denen eine als Extremitätenbandage ausgebildete Bandage hergestellt werden soll. Die dargestellten Teile umfassen einen Gestrickkörper 1, eine Pelotte 2 und eine Pelottenabdeckung 3. Die Pelottenabdeckung 3 besteht aus einem Abdecktextil 4 und einem rückseitig an das Abdecktextil 4 laminiertem Schmelzklebevlies 5.

Der Gestrickkörper 1, die Pelotte 2 und die Pelottenabdeckung 3 sind alle dehnbar und weisen jeweils ihre größte Dehnbarkeit in Richtung der in Figur 1 dargestellten Pfeile auf. Diese Richtung ist die Umfangsrichtung der aus dem Gestrickkörper 1, der Pelotte 2 und der Pelottenabdeckung 3 herzustellenden Bandage. Die Dehnbarkeit des Gestrickkörpers 1 ist auf bekannt Weise durch ein hierfür geeignetes Strickmuster in Kombination mit geeigneten und gegebenenfalls dehnbaren und elastischen Materialien hergestellt. Die neue Kombination mit der besonders dehnbaren Pelotte 2 und der besonders dehnbaren Pelottenabdeckung 3 ermöglicht eine besonders große Dehnbarkeit der herzustellenden Bandage. Von entscheidender Bedeutung ist hierbei das Schmelzklebevlies 5 der Pelottenabdeckung 3, welches im Gegensatz zu herkömmlicherweise bei der Herstellung von Bandagen verwendeten geschlossenen Klebstoffschichten offen ist und dadurch auch nach dem Verkleben der Pelottenabdeckung 3 mit dem Gestrickkörper 1 und der Pelotte 2 eine große Dehnbarkeit ermöglicht. Das Flächengewicht des Schmelzklebevlieses 5 liegt dabei deutlich unter dem Flächengewicht bekannter geschlossener Schmelzkleberschichten. Es hat sich gezeigt, dass damit dennoch eine dauerhaft stabile Klebeverbindung zwischen der Pelottenabdeckung 3 und dem Gestrickkörper 1 sowie der Pelotte 2 hergestellt werden kann.

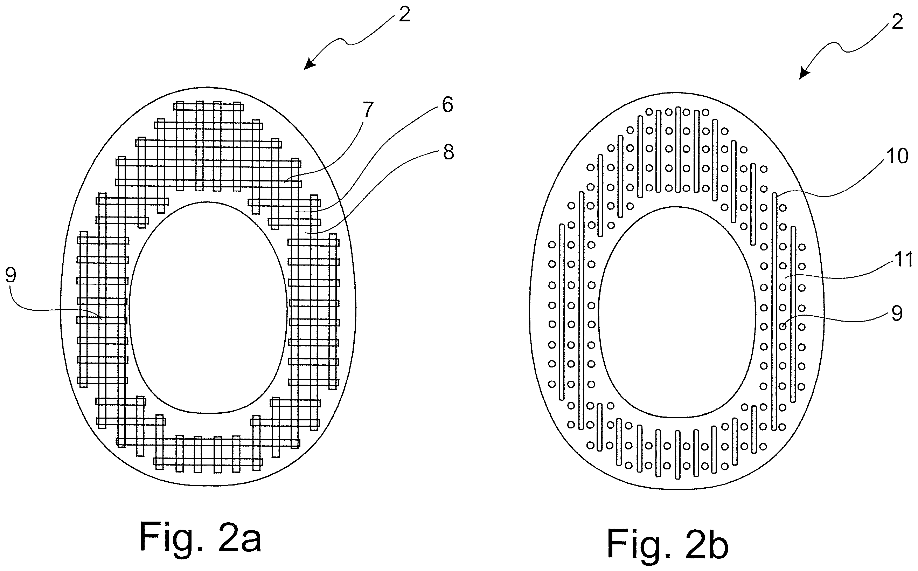

In den Figuren 2a und 2b ist eine ringförmig ausgebildete Pelotte 2 dargestellt, die sich zumindest in ihrer Form von der Pelotte 2 von Figur 1 unterscheidet. Gleiche Bezugszeichen bezeichnen in allen Figuren gleiche oder einander entsprechende Teile. Die Pelotte 2 von den Figuren 2a und 2b ist als Patella-Pelotte ausgebildet und zur Abstützung im Kniebereich und der Patellasehne vorgesehen. In Figur 2a ist die Innenseite, also die bei der fertiggestellten Bandage der Pelottenabdeckung 3 und dem Körper zugewandte Seite der Pelotte 2 zu sehen. In Figur 2b ist die Außenseite, also die bei der fertiggestellten Bandage dem Gestrickkörper 1 zugewandte und dem Körper abgewandte Seite der Pelotte 2 zu sehen.

An der in Figur 2a sichtbaren Innenseite der Pelotte 2 sind Lüftungskanäle 6 und 7 ausgebildet, wobei die Lüftungskanäle 6 parallel zueinander angeordnet sind und eine erste Gruppe der Lüftungskanäle 6 und 7 bilden und wobei die Lüftungskanäle 7 ebenfalls parallel zueinander angeordnet sind und eine zweite, im rechten Winkel zur ersten Gruppe verlaufende Gruppe der Lüftungskanäle 6 und 7 bilden. Zwischen den Lüftungskanälen 6 und 7 ist eine Vielzahl quadratischer Anlagekörper 8 ausgebildet. In den Kreuzungsbereichen der Lüftungskanäle 6 mit den Lüftungskanälen 7 sind Durchbrüche 9 ausgebildet, die dazu vorgesehen und ausgebildet sind, Flüssigkeit, insbesondere Körperschweiß, über Verdunstung von der Innenseite zur Außenseite zu transportieren. Andere Anordnungen von Lüftungskanälen 6, 7 sind denkbar, bei denen sich beispielsweise drei Gruppen von Lüftungskanälen 6, 7 in einem Winkel von 60° schneiden, so dass dreieckige Anlagekörper 8 ausgebildet werden.

An der in Figur 2b sichtbaren Außenseite der Pelotte 2 sind Außenkanäle 10 ausgebildet. Diese sind parallel zueinander ausgerichtet und im Ergebnis parallel zu der ersten Gruppe von Lüftungskanälen 6 auf der Innenseite der Pelotte 2 ausgerichtet. Zwischen den Außenkanälen 8 sind Wandbereiche 11 vorhanden. In diesen Wandbereichen 11 münden die Durchbrüche 9, die sich von der Innenseite zur Außenseite der Pelotte 2 erstrecken. Durch die versetzte Anordnung der Lüftungskanäle 6 und der Außenkanäle 10 ergibt sich eine Ziehharmonikastruktur, durch die eine besonders hohe Elastizität der Pelotte 2 in einer Richtung senkrecht zu den Lüftungskanälen 6 und den Außenkanälen 10 erreicht wird.

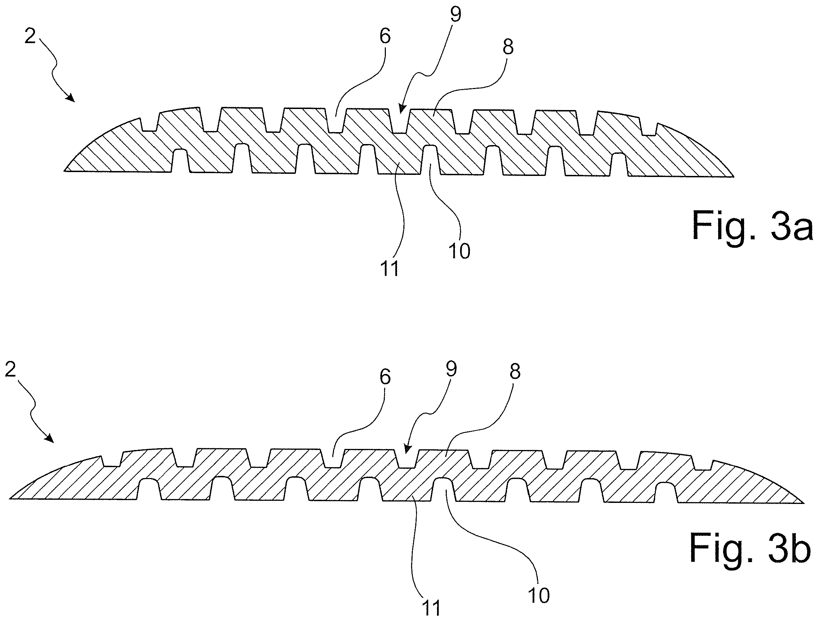

In den Figuren 3a und 3b ist jeweils ein Querschnitt durch die als Patella-Pelotte ausgebildete Pelotte 2 von den Figuren 2a und 2b dargestellt. Die obere Seite ist die Innenseite, die von der Pelottenabdeckung 3 bedeckt und körperseitig getragen wird. Die untere Seite ist die Außenseite, die vom Gestrickkörper 1 bedeckt und auf der vom Körper abgewandten Seite getragen wird. Auf der Innenseite sind die Lüftungskanäle 6 der ersten Gruppe und auf der Außenseite die Außenkanäle 10 zu erkennen. In Figur 3b ist die Pelotte 2 gegenüber der Darstellung von Figur 3a gedehnt dargestellt. Die besonders große Dehnbarkeit der Pelotte 2 wird durch die mittels der Lüftungskanäle 6 und der Außenkanäle 10 ausgebildeten Ziehharmonikastruktur bewirkt, wie aus der Gegenüberstellung der Figuren 3a und 3b besonders deutlich wird.

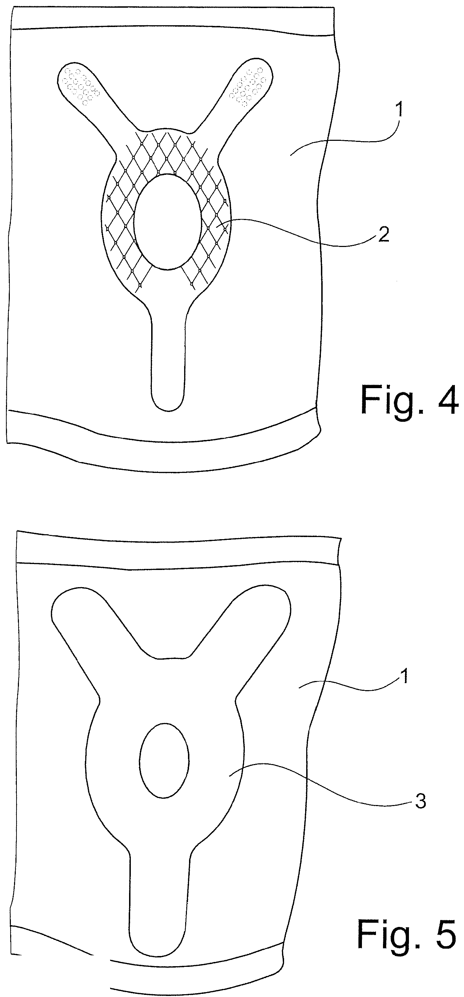

Figur 4 zeigt den Gestrickkörper 1 und die Pelotte 1 von Figur 1, also im Prinzip die Anordnung von Figur 1 ohne die in Figur 1 dargestellte Pelottenabdeckung 3. Die Pelotte 2 liegt mit ihrer Außenseite auf dem Gestrickkörper 1 auf und ist mit ihrer Innenseite dem Betrachter zugewandt. Die Anordnung der Lüftungskanäle 6 und 7 und der Außenkanäle 10 ist bevorzugt entsprechend der Anordnung bei der Pelotte 2 von den Figuren 2 und 3.

In Figur 5 ist im Unterschied zur Anordnung von Figur 4 zusätzlich die Pelottenabdeckung 3 auf die Pelotte 2 aufgelegt, so dass die Pelotte 2 vollständig von der Pelottenabdeckung 3 bedeckt ist. Die Pelottenabdeckung 3 steht zudem in ihren Randbereichen gegenüber der Pelotte 2 über.

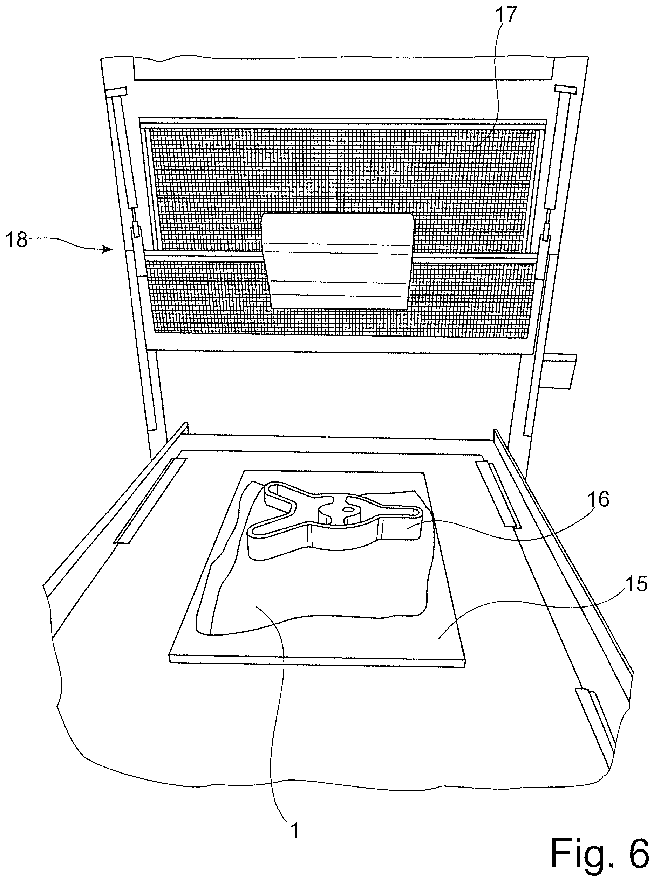

In Figur 6 sind der Gestrickkörper 1, die Pelotte 2 und die Pelottenabdeckung 3 gemäß Ihrer Anordnung von Figur 5 zwischen einer flächigen Elektrode 15, auf der der Gestrickkörper 1 aufliegt, und einer aufgelegten Elektrode 16, die auf der Pelottenabdeckung 3 aufliegt, angeordnet. Die aufgelegte Elektrode 16 ist in ihren Konturen derart an die Konturen der Pelotte 2 und der Pelottenabdeckung 3 angepasst, dass die aufgelegte Elektrode 16 im Prinzip nur in den gegenüber der Pelotte 2 vorstehenden Randbereichen der Pelottenabdeckung 3 auf der Pelottenabdeckung 3 aufliegt.

Die Elektroden 15 und 16 sind zusammen mit einem im Hintergrund dargestellten Käfig 17 Teile einer Hochfrequenzeinrichtung 18, mittels der eine Hochfrequenzspannung an die Elektroden 15 und 16 angelegt werden kann, um das im Bereich zwischen den Elektroden 15 und 16 angeordnete Schmelzklebevlies 5 zu erhitzen und damit zeitweilig soweit zu verflüssigen. Dadurch kann eine Klebeverbindung zwischen dem Gestrickkörper 1 und der Pelottenabdeckung 3 in den Randbereichen der Pelottenabdeckung 3 sowie eine Klebeverbindung zwischen der Pelottenabdeckung 3 und der Pelotte 2 im Bereich dazwischen hergestellt werden. Hierfür wird die Anordnung aus dem Gestrickkörper 1, der Pelotte 2, der Pelottenabdeckung 3 und der Elektroden 15 und 16 in den Käfig 17 eingeschoben. Außerdem wird die aufgelegte Elektrode 16 in Richtung der flächigen Elektrode 15 während des Erhitzens des Schmelzklebevlieses 5 und anhaltend noch so lange auf die Pelottenabdeckung 3 gedrückt, bis der Schmelzkleber des Schmelzklebevlieses wieder ausreichend abgekühlt und ausgehärtet ist.

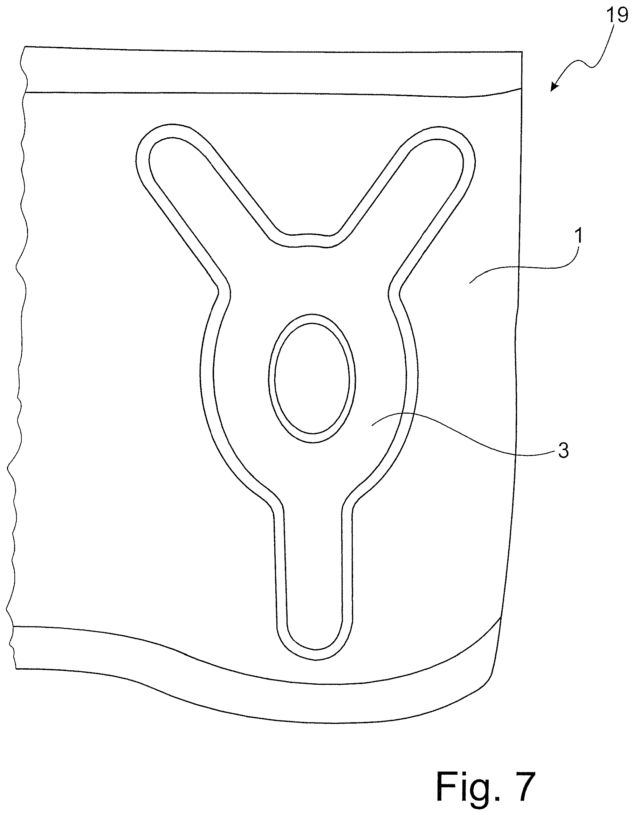

Figur 7 zeigt das Ergebnis des mittels der Hochfrequenzeinrichtung 18 von Figur 6 getätigten Schweißvorgangs oder Klebevorgangs, nachdem die aufgelegte Elektrode 16 wieder von der Pelottenabdeckung 3 entfernt wurde, nämlich die fertiggestellte und hier erstmals mit einem Bezugszeichen bezeichnete Bandage 19. Bei der Bandage 19 ist die Pelottenabdeckung 3 fest und auch optisch hochwertig erscheinend in ihren Randbereichen mit dem Gestrickkörper 1 verbunden, so dass die Pelotte 2 vollständig von dem Gestrickkörper 1 und der Pelottenabdeckung 3 umschlossen ist. Die Pelotte 2 ist außerdem mittels des wieder gehärteten Schmelzklebers des Schmelzklebevlieses 5 am Abdecktextil 4 der Pelottenabdeckung 3 fixiert.

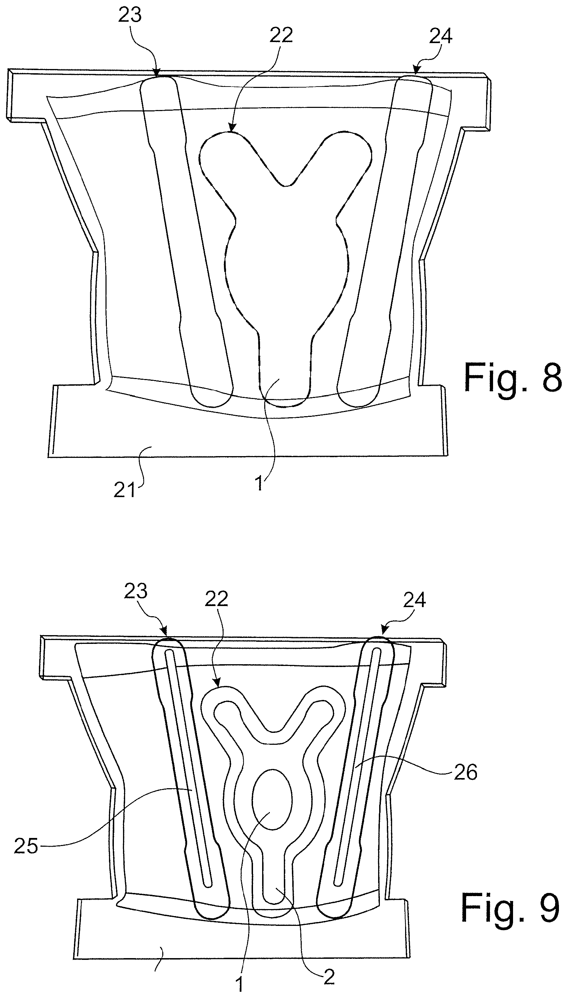

In den Figuren 8, 9, 10 und 11 ist die Positionierung der Pelotte 2, der Pelottenabdeckung 3 und der aufgelegten Elektrode 16 auf dem Gestrickkörper 1 mithilfe einer Schablone 21 dargestellt. Die Schablone 21 ist transparent ausgebildet und in Figur 8 auf den Gestrickkörper 1 aufgelegt. Neben einer zentralen Aussparung 22 in der Größe der Pelottenabdeckung 3 sind eine erste seitliche Aussparung 23 und eine zweite seitliche Aussparung 24 vorgesehen.

In Figur 9 ist die Pelotte 2 mit Abstand zu den Rändern der zentralen Aussparung 22 auf den Gestrickkörper 1 aufgelegt. Außerdem sind in die seitlichen Aussparungen 23 und 24 ebenfalls mit Abstand zu deren Konturen eine erste Flachspiralfederschiene 25 und eine zweite Flachspiralfederschiene 26 aufgelegt. Die Flachspiralfederschienen 25 und 26 sorgen bei der fertiggestellten Bandage 19 für zusätzliche Stabilität.

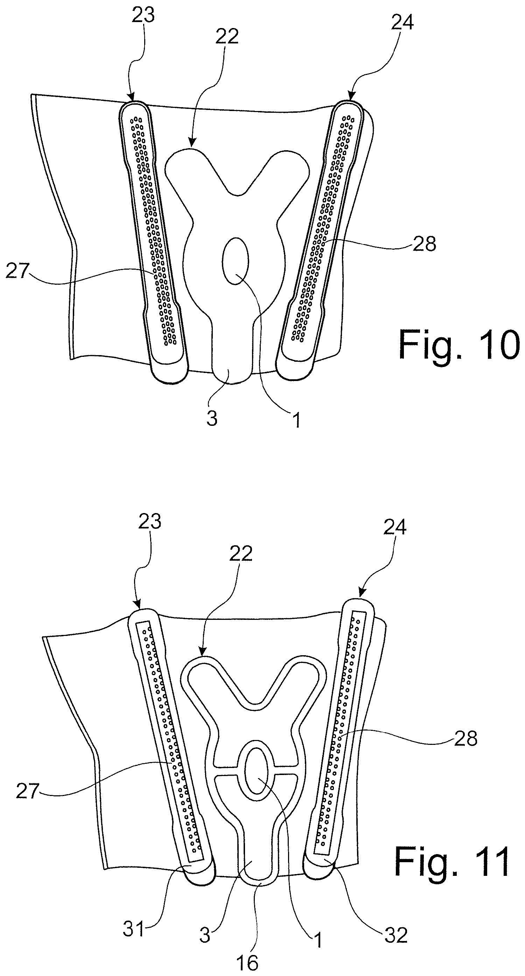

In Figur 10 sind mithilfe der Schablone 21 die Pelottenabdeckung 3 auf der Pelotte 2 sowie eine erste Schienenabdeckung 27 auf der ersten Flachspiralfederschiene 25 und eine zweite Schienenabdeckung 28 auf der zweiten Flachspiralfederschiene 26 positioniert. Die Schienenabdeckungen 27 und 28 sind bevorzugt entsprechend der Pelottenabdeckung 3 mit dem Abdecktextil 4 und dem Schmelzklebevlies 3 aufgebaut.

Figur 11 zeigt zusätzlich zu der Anordnung gemäß Figur 10 die mittels der Schablone 21 positionierte und auf die Pelottenabdeckung 3 aufgelegte Elektrode 16 sowie eine erste seitliche Elektrode 31 und eine zweite seitliche Elektrode 32. Die seitlichen Elektroden 31 und 32 sind ebenfalls mittels der Schablone 21 positioniert und auf die erste Schienenabdeckung 27 sowie die zweite Schienenabdeckung 28, insbesondere deren über die Konturen der Flachspiralfederschienen 25 und 26 überstehende Ränder, aufgelegt. In der Hochfrequenzeinrichtung 18 können die Schienenabdeckungen 27 und 28 somit durch Aufschmelzen ihrer Klebeschicht, insbesondere Schmelzklebevliesschicht, mit den Flachspiralfederschienen 25 und 26 und an ihren Rändern ebenfalls mit dem Gestrickkörper 1 verbunden werden. Die Flachspiralfederschienen 25 und 26 sind nachfolgend entsprechend der Anordnung der Pelotte 2 zwischen der Pelottenabdeckung 3 und dem Gestrickkörper 1 vollständig von jeweils einer der Schienenabdeckungen 27 und 28 und dem Gestrickkörper 1 umschlossen.

## Patentansprüche

1. Verfahren zum Befestigen einer Pelotte (2) an einem elastischen Gestrickkörper (1) einer herzustellenden Bandage (19), insbesondere Extremitätenbandage,
bei dem die Pelotte (2) an den Gestrickkörper (1) angelegt wird,
bei dem eine Pelottenabdeckung (3) auf der dem Gestrickkörper (1) abgewandten Seite der Pelotte (2) an die Pelotte (2) angelegt wird und
bei dem die Pelottenabdeckung (3) rings um die Kontur der Pelotte (2) mit dem Gestrickkörper (1) verbunden wird, so dass die Pelotte (2) vollständig von dem Gestrickkörper (1) und der Pelottenabdeckung (3) umschlossen wird, wobei die Pelottenabdeckung (3) zumindest rings um die Kontur der Pelotte (2) gegen den Gestrickkörper (1) gedrückt und zum Verbinden mit dem Gestrickkörper (1) ein offenes Schmelzklebevlies (5), mit dem die Pelottenabdeckung (3) ausgestattet ist, währenddessen erhitzt wird und die Pelotte (2) mit der Richtung ihrer größten Dehnbarkeit, in der die Pelotte (2) insbesondere um ein Mehrfaches dehnbarer ist als senkrecht zu dieser Richtung, in Umfangsrichtung der herzustellenden Bandage (19) zwischen dem Gestrickkörper (1) und der Pelottenabdeckung (3) angeordnet wird,
**dadurch gekennzeichnet,**
**dass** die Pelotte (2) Außenkanäle (10) auf der Seite, die dem Gestrickkörper (1) zugewandt ist, aufweist und dass die Pelotte (2) Lüftungskanäle (6, 7) auf der Seite, die der Pelottenabdeckung (3) zugewandt ist, aufweist und dass die Lüftungskanäle (6) zumindest teilweise parallel zu den Außenkanälen (10) angeordnet sind.

2. Verfahren nach Anspruch 1, **dadurch gekennzeichnet, dass** die Außenkanäle (10) und die parallel zu den Außenkanälen (10) angeordneten Lüftungskanäle (6) abwechselnd zueinander, insbesondere zur Ausbildung einer Art Ziehharmonika-Struktur, angeordnet sind.

3. Verfahren nach einem der Ansprüche 1 oder 2, **dadurch gekennzeichnet, dass** die Pelotte (2) Durchbrüche (9) aufweist, die in die Lüftungskanäle (6, 7) münden.

4. Verfahren nach einem der vorhergehenden Ansprüche, **dadurch gekennzeichnet, dass** der Gestrickkörper (1) und die Pelotte (2) und die Pelottenabdeckung (3) mit dem Schmelzklebevlies (5) jeweils dehnbar sind und mit der Richtung ihrer jeweiligen größten Dehnbarkeit parallel zueinander angeordnet und miteinander verbunden werden.

5. Verfahren nach einem der vorhergehenden Ansprüche, **dadurch gekennzeichnet, dass** der Gestrickkörper (1) schlauchförmig ausgebildet ist und dass die Richtung der größten Dehnbarkeit des Gestrickkörpers (1) seine Umfangsrichtung ist.

6. Verfahren nach einem der vorhergehenden Ansprüche, **dadurch gekennzeichnet, dass** die Pelottenabdeckung (3) aus einem Abdecktextil (4), insbesondere elastischem Frottee, und dem Schmelzklebevlies (5) besteht, wobei das Abdecktextil (4) entweder bereits mit dem Schmelzklebevlies (5) verbunden, insbesondere laminiert, an die Pelotte (2) angelegt wird oder erst dann mit dem Schmelzklebevlies (5) verbunden wird, wenn die Pelottenabdeckung (3) mit dem Gestrickkörper (1) verbunden wird.

7. Verfahren nach einem der vorhergehenden Ansprüche, **dadurch gekennzeichnet, dass** das Schmelzklebevlies (5) kapazitiv in einem elektrischen Wechselfeld erwärmt wird,

8. Verfahren nach einem der vorhergehenden Ansprüche, **dadurch gekennzeichnet, dass** wenigstens eine Elektrode (16) auf die Pelottenabdeckung (3) aufgelegt wird und dass das Schmelzklebevlies (5) mit Hilfe der auf die Pelottenabdeckung (3) aufgelegten Elektrode (16) kapazitiv erwärmt wird.

9. Verfahren nach einem der vorhergehenden Ansprüche, **dadurch gekennzeichnet, dass** das Schmelzklebevlies (5) ein Flächengewicht von mehr als 30 g/m² und weniger als 100 g/m², insbesondere ein Flächengewicht von etwa 60 g/m², aufweist.

10. Verfahren zum Herstellen einer Bandage (19), insbesondere Extremitätenbandage, mit einem elastischen Gestrickkörper (1) und einer am Gestrickkörper (1) befestigten Pelotte (2), wobei die Pelotte (2) mit dem Verfahren nach einem der vorhergehenden Ansprüche am Gestrickkörper (1) befestigt wird.

11. Gestrickkörper (1) mit daran befestigter Pelotte (2),
bei dem die Pelotte (2) am Gestrickkörper (1) angelegt ist,
bei dem eine Pelottenabdeckung (3) auf der dem Gestrickkörper (1) abgewandten Seite der Pelotte (2) an die Pelotte (2) angelegt ist und
bei dem die Pelottenabdeckung (3) rings um die Kontur der Pelotte (2) mit dem Gestrickkörper (1) verbunden ist, so dass die Pelotte (2) vollständig von dem Gestrickkörper (1) und der Pelottenabdeckung (3) umschlossen ist,
**dadurch gekennzeichnet,**
**dass** die Pelotte (2) mit dem Verfahren nach einem der Ansprüche 1 bis 9 mittels eines offenen Schmelzklebevlieses (5), mit dem die Pelottenabdeckung (3) ausgestattet ist, mit dem Gestrickkörper (1) verbunden ist.

12. Bandage (19), insbesondere Extremitätenbandage, die einen schlauchförmigen Gestrickkörper (1) mit daran befestigter Pelotte (2) nach Anspruch 11 aufweist.

## Claims

1. A method for fastening a truss pad (2) to an elastic knitted body (1) of a dressing (19) which is to be produced, in particular an extremity dressing,
in which the truss pad (2) is placed onto the knitted body (1),
in which a truss pad cover (3) is placed onto the truss pad (2) on the side of the truss pad (2) facing away from the knitted body (1), and
in which the truss pad cover (3) is connected to the knitted body (1) all around the contour of the truss pad (2), so that the truss pad (2) is entirely surrounded by the knitted body (1) and the truss pad cover (3), wherein the truss pad cover (3) is pressed against the knitted body (1) at least all around the contour of the truss pad (2) and for connecting to the knitted body (1), an open hot-melt adhesive nonwoven fabric (5), with which the truss pad cover (3) is equipped, is heated during the process, and the truss pad (2) with the direction of its greatest stretchability, in which the truss pad (2) is more stretchable in partiuclar by a multiple than perpendicularly to this direction, is arranged between the knitted body (1) and the truss pad cover (3) in circumferential direction of the dressing (19) which is to be produced,
**characterized in that**
the truss pad (2) has outer channels (10) on the side which faces the knitted body (1), and that the truss pad (2) has ventilation channels (6, 7) on the side which faces the truss pad cover (3), and that the ventilation channels (6) are arranged at least partially parallel to the outer channels (10).

2. The method according to Claim 1, **characterized in that** the outer channels (10) and the ventilation channels (6) arranged parallel to the outer channels (10) are arranged alternating with respect to one another, in particular for the formation of a type of concertina structure.

3. The method according to one of Claims 1 or 2, **characterized in that** the truss pad (2) has apertures (9) which open into the ventilation channels (6, 7).

4. The method according to one of the preceding claims, **characterized in that** the knitted body (1) and the truss pad (2) and the truss pad cover (3) with the hot-melt adhesive nonwoven fabric (5) are respectively stretchable and are arranged parallel to one another with the direction of their respective greatest stretchability and connected to one another.

5. The method according to one of the preceding claims, **characterized in that** the knitted body (1) is configured in a tube-shaped manner, and that the direction of the greatest stretchability of the knitted body (1) is its circumferential direction.

6. The method according to one of the preceding claims, **characterized in that** the truss pad cover (3) consists of a covering textile (4), in particular elastic terry cloth, and of the hot-melt adhesive nonwoven fabric (5), wherein the covering textile (4) either already connected, in particular laminated, to the hot-melt adhesive nonwoven fabric (5), is placed onto the truss pad (2), or is only connected to the hot-melt adhesive nonwoven fabric (5) when the truss pad cover (3) is connected to the knitted body (1).

7. The method according to one of the preceding claims, **characterized in that** the hot-melt adhesive nonwoven fabric (5) is heated capacitively in an alternating electric field.

8. The method according to one of the preceding claims, **characterized in that** at least one electrode (16) is placed onto the truss pad cover (3) and that the hot-melt adhesive nonwoven fabric (5) is heated capacitively by means of the electrode (16) which is placed onto the truss pad cover (3).

9. The method according to one of the preceding claims, **characterized in that** the hot-melt adhesive nonwoven fabric (5) has a weight per unit area of more than 30 g/m² and less than 100 g/m², in particular a weight per unit area of approximately 60 g/m².

10. A method for producing a dressing (19), in particular an extremity bandage, with an elastic knitted body (1) and with a truss pad (2) fastened to the knitted body (1), wherein the truss pad (2) is fastened to the knitted body (1) by the method according to one of the preceding claims.

11. A knitted body (1) with truss pad (2) fastened thereto,
in which the truss pad (2) is placed on the knitted body (1),
in which a truss pad cover (3) is placed onto the truss pad (2) on the side of the truss pad (2) facing away from the knitted body (1), and
in which the truss pad cover (3) is connected to the knitted body (1) all around the contour of the truss pad (2), so that the truss pad (2) is entirely surrounded by the knitted body (1) and the truss pad cover (3),
**characterized in that**
the truss pad (2) is connected to the knitted body (1) by the method according to one of Claims 1 to 9 by means of an open hot-melt adhesive nonwoven fabric (5) with which the truss pad cover (3) is equipped.

12. A dressing (19), in particular an extremity dressing, which has a tube-shaped knitted body (1) with truss pad (2) fastened thereto according to Claim 11.

## Revendications

1. Procédé de fixation d'un tampon en treillis (2) sur un corps tricoté élastique (1) d'un pansement (19) à fabriquer, en particulier pansement d'extrémité,
pour lequel le tampon en treillis (2) est appliqué au corps tricoté (1),
pour lequel une couverture de tampon en treillis (3) est appliquée au tampon en treillis (2) sur le côté du tampon en treillis (2) éloigné du corps tricoté (1) et pour lequel la couverture de tampon en treillis (3) est reliée au corps tricoté (1) tout autour du contour du tampon en treillis (2) de telle manière que le tampon en treillis (2) est complètement entouré par le corps tricoté (1) et la couverture de tampon en treillis (3), sachant que la couverture de tampon en treillis (3) est appuyée au moins tout autour du contour du tampon en treillis (2) contre le corps tricoté (1) et un non tissé à adhésif thermofusible (5) ouvert, dont la couverture de tampon en treillis (3) est dotée, est chauffé pendant ce temps pour la liaison avec le corps tricoté (1) et le tampon en treillis (2) est disposé dans la direction périphérique du pansement (19) à fabriquer entre le corps tricoté (1) et la couverture de tampon en treillis (3), dans la direction de sa plus grande expansibilité, dans laquelle le tampon en treillis (2) peut être expansé en particulier plusieurs fois perpendiculairement à cette direction,
**caractérisé en ce que**
le tampon en treillis (2) comporte des conduits extérieurs (10) sur le côté qui est tourné vers le corps tricoté (1) et **en ce que** le tampon en treillis (2) comporte des conduits d'aération (6, 7) sur le côté, qui est tourné vers la couverture de tampon en treillis (3) et **en ce que** les conduits d'aération (6) sont disposés au moins en partie parallèlement aux conduits extérieurs (10).

2. Procédé selon la revendication 1, **caractérisé en ce que** les conduits extérieurs (10) et les conduits d'aération (6) disposés parallèlement aux conduits extérieurs (10) sont disposés en alternance les uns par rapport aux autres, en particulier pour former un type de structure en accordéon.

3. Procédé selon l'une quelconque des revendications 1 ou 2, **caractérisé en ce que** le tampon en treillis (2) comporte des passages (9), qui débouchent dans les conduits d'aération (6,7).

4. Procédé selon l'une quelconque des revendications précédentes, **caractérisé en ce que** le corps tricoté (1) et le tampon en treillis (2) et la couverture de tampon en treillis (3) peuvent être respectivement expansés avec le non tissé à adhésif thermofusible (5) et sont disposés parallèlement l'un par rapport à l'autre et reliés entre eux dans la direction de leur plus grande expansibilité respective.

5. Procédé selon l'une quelconque des revendications précédentes, **caractérisé en ce que** le corps tricoté (1) est constitué en forme tubulaire et **en ce que** la direction de la plus grande expansibilité du corps tricoté (1) est sa direction périphérique.

6. Procédé selon l'une quelconque des revendications précédentes, **caractérisé en ce que** la couverture de tampon en treillis (3) est composée d'un textile de couverture (4), en particulier de tissu éponge élastique et du non tissé à adhésif thermofusible (5), sachant que le textile de couverture (4) est appliqué au tampon en treillis (2), soit déjà relié, en particulier contrecollé au non tissé à adhésif thermofusible (5), soit n'est ensuite relié au non tissé à adhésif thermofusible (5) que lorsque la couverture de tampon en treillis (3) est reliée au corps tricoté (1).

7. Procédé selon l'une quelconque des revendications précédentes, **caractérisé en ce que** le non tissé à adhésif thermofusible (5) est chauffé de façon capacitive dans un champ alternatif électrique.

8. Procédé selon l'une quelconque des revendications précédentes, **caractérisé en ce qu'**au moins une électrode (16) est posée sur la couverture de tampon en treillis (3) et **en ce que** le non tissé à adhésif thermofusible (5) est chauffé de façon capacitive à l'aide de l'électrode (16) posée sur la couverture de tampon en treillis (3).

9. Procédé selon l'une quelconque des revendications précédentes, **caractérisé en ce que** le non tissé à adhésif thermofusible (5) comporte un grammage de plus de 30 g/m² et de moins de 100 g/m², en particulier un grammage d'environ 60g/m².

10. Procédé de fabrication d'un pansement (19), en particulier d'un pansement d'extrémité, avec un corps tricoté élastique (1) et un tampon en treillis (2) fixé sur le corps tricoté (1), sachant que le tampon en treillis (2) est fixé sur le corps tricoté (1) avec le procédé selon l'une quelconque des revendications précédentes.

11. Corps tricoté (1) sur lequel est fixé un tampon en treillis (2),
pour lequel le tampon en treillis (2) est appliqué au corps tricoté (1),
pour lequel une couverture de tampon en treillis (3) est appliquée au tampon en treillis (2) sur le côté du tampon en treillis (2) éloigné du corps tricoté (1) et pour lequel la couverture de tampon en treillis (3) est reliée tout autour du contour du tampon en treillis (2) au corps tricoté (1) de telle manière que le tampon en treillis (2) est complètement entouré par le corps tricoté (1) et la couverture de tampon en treillis (3),
**caractérisé en ce que**
le tampon en treillis (2) est relié au corps tricoté (1) avec le procédé selon l'une quelconque des revendications 1 à 9 au moyen d'un non tissé à adhésif thermofusible (5) ouvert dont la couverture de tampon en treillis (3) est dotée.

12. Pansement (19), en particulier pansement d'extrémité, qui comporte un corps tricoté (1) tubulaire sur lequel est fixé le tampon en treillis (2) selon la revendication 11.
